# EUROPEAN PATENT SPECIFICATION

(11) **EP 3 429 589 B1**
(45) Date of publication and mention of the grant of the patent: **14.12.2022**
(21) Application number: 17766004.0
(22) Date of filing: 17.03.2017
(51) Int. Cl.: A61K 31/506, C07D 401/14, A61P 35/02, A61K 9/20, A61K 9/48

(54) **PHARMACEUTICAL COMPOSITION OF NILOTINIB**
PHARMAZEUTISCHE ZUSAMMENSETZUNG AUS NILOTINIB
COMPOSITION PHARMACEUTIQUE DU NILOTINIB

(30) Priority: 17.03.2016 IN 201621009437
(43) Date of publication of application: 23.01.2019
(73) Proprietor: Sun Pharmaceutical Industries Ltd, 400063 Mumbai Maharashtra (IN)
(72) Inventor: JAHAGIRDAR, Harshal, Gujarat Baroda 390020 (IN); JADHAV, Bhushan, Gujarat Baroda 390020 (IN); KULKARNI, Amol, Gujarat Baroda 390020 (IN); KULKARNI, Shirish, Gujarat Baroda 390020 (IN); THENNATI, Rajamannar, Gujarat Baroda 390020 (IN)
(74) Representative: Harrison IP Limited
(86) International application number: PCT/IN2017/050098
(87) International publication number: WO 2017/158625

(56) References cited:
- WO-A1-2015/049371
- WO-A2-2016/024289
- KANTARJIAN HAGOP ET AL: "NILOTINIB IN IMATINIB-RESISTANT CML AND PHILADELPHIA CHROMOSOME-POSITIVE", NEW ENGLAND JOURNAL OF MEDICINE, THE - NEJM, MASSACHUSETTS MEDICAL SOCIETY, US, vol. 354, no. 24, 15 June 2006 (2006-06-15), pages 2542-2551, XP009072571, ISSN: 1533-4406, DOI: 10.1056/NEJMOA055104
- TANAKA C ET AL: "Clinical Pharmacokinetics of the BCR-ABL Tyrosine Kinase Inhibitor Nilotinib", CLINICAL PHARMACOLOGY AND THERAPEUTICS, NATURE PUBLISHING GROUP, US, vol. 87, no. 2, 18 November 2009 (2009-11-18), pages 197-203, XP009142210, ISSN: 0009-9236

## Description

### FIELD OF THE INVENTION

The present invention relates to a dosage form having a composition comprising nilotinib butanedisulphonate (2:1) or nilotinib butanedisulphonate (1:1) for use in treating resistant or intolerant Philadelphia chromosome positive chronic myelogenous leukaemia. A composition comprising nilotinib butanedisulphonate (2:1) or nilitinib butanedisulphonate (1:1) for use in treating newly diagnosed Philadelphia chromosome positive chronic myeloid leukaemia is also provided.

### BACKGROUND OF THE INVENTION

Nilotinib, 4-methyl-N-[3-(4-methyl-1H-imidazol-1-yl)-5-(trifluoromethyl)phenyl]-3-[[4-(3-pyridinyl)-2-pyrimidinyl]amino]-benzamide, having the following formula

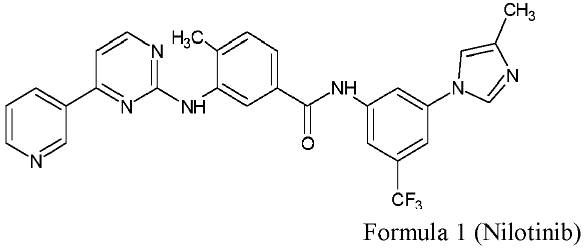

It is marketed under the name Tasigna^{®} in US and Europe. Tasigna^{®} is available as hard capsules containing nilotinib hydrochloride equivalent to 150 mg and 200 mg of Nilotinib, for the treatment of adult patients with newly diagnosed Philadelphia chromosome positive chronic myeloid leukemia (Ph+ CML) in chronic phase. Tasigna^{®} is also indicated for the treatment of chronic phase and accelerated phase Philadelphia chromosome positive chronic myelogenous leukemia (Ph+ CML) in adult patients resistant or intolerant to prior therapy that included imatinib. Nilotinib, particularly, its hydrochloride is poorly water soluble and is reported to be difficult to formulate and deliver. It is prescribed as 300 mg twice daily (600 mg daily dose) for treatment of newly diagnosed Philadelphia chromosome positive chronic myeloid leukemia and 400 mg twice daily (800 mg twice daily) for treatment resistant or intolerant Philadelphia chromosome positive chronic myelogenous leukemia. Further, the bioavailability is increased when given with a meal. (Castagnetti et al; Hematology Meeting Reports, 2008; 2 (5): 22-26). Compared to the fasted state, the systemic exposure (AUC) is reported to increase by 82 % when the dose is given 30 minutes after a high fat meal. The pack insert of Tasigna^{®} reveals that the patients are instructed to take the capsules twice daily with 12 hours apart time. Further, the capsules need to be necessarily administered on an empty stomach. No food should be consumed for at least 3 hours before the dose is taken and for at least 1 hour after the dose is taken.

US20150273070 discloses a dosage form comprising amorphous form of organic acid salts of nilotinib. The patent application describes the composition having nilotinib hydrochloride along with organic acids such as citric acid, acetic acid, succinic acid in the composition. The dosage form comprising amorphous form of organic acid salts of nilotinib increased the bioavailability in dogs and use of the organic acid was found to suppress the food effect on bioavailability.

WO2016/024289 discloses nilotinib butanedisulphonate for use in the treatment of leukemia including chronic myeloid leukemia.

WO2015/049371 discloses the treatment of chronic myeloid leukemia or Philadelphia chromosome-positive acute lymphoblastic leukemia by administration of a tyrosine kinase inhibitor such as nilotinib typically at a dosage of 0.01 to about 500mg.

Oral administration of nilotinib at does ranging from 50 mg to 1200mg once daily, and at 400mg and 600mg twice daily, for use in the treatment of resistant Philadelphia chromosome positive chronic myelogenous leukemia is disclosed in Kantarjian Hagop et al; New England Journal of Medicine, NELM Massachusetts medical society; vol 354, no 24, 15 June 2002, pages 2542-2551.

The administration of nilotinib at does ranging from 50 mg to 1200mg once daily, for use in the treatment of Philadelphia chromosome positive chronic myelogenous leukemia is disclosed in Tanaka et al Clinical Pharmacology and Therapeutics, Nature publishing group, vol 87, no 2, 1 February 201: pages 197-203.

Commercially available preparations of nilotinib pose risk of adverse effects particularly because they are required to be administered on empty stomach, however, if the patient ingests the tablets of nilotinib with or after meals, particularly, high fat meals, the rate and extent of absorption (area under the plasma profile curve and the Cₘₐₓ) are increased by 82 % and 112 %, respectively (Castagnetti et al; Hematology Meeting Reports, 2008; 2 (5); 22-26). The present inventors have found a method to reduce such risk. The inventors have found novel dosage form having a composition that uses nilotinib butane disulphonate and provides improved oral bioavailability of nilotinib while also ensuring a reduced food effect on oral bioavailability.).

### SUMMARY OF THE INVENTION

The present invention provides a pharmaceutical dosage form having a composition comprising nilotinib butanedisulphonate (2:1) or nilotinib butanedisulphonate (1:1) for use in treating resistant or intolerant Philadelphia chromosome positive chronic myelogenous leukaemia, comprising orally administering to a patient in need thereof daily doses of nilotinib in the range from 300 mg to 500 mg

The present invention also provides a dosage form having a composition comprising nilotinib butanedisulphonate (2:1) or nilotinib butanedisulphonate (1:1) for use in the treatment of newly diagnosed Philadelphia chromosome positive chronic myeloid leukemia at a total daily dose in the range from 300 mg to 375 mg.

### BRIEF DESCRIPTION OF THE DRAWINGS

Figure 1 shows a XRD pattern of a crystalline Form II of 2:1 salt of nilotinib butanedisulfonic acid having a powder X-ray diffraction pattern having powder X-ray diffraction peaks at 5.9, 8.1, 26.3 and 26.9 ± 0.2 degrees 2-theta.

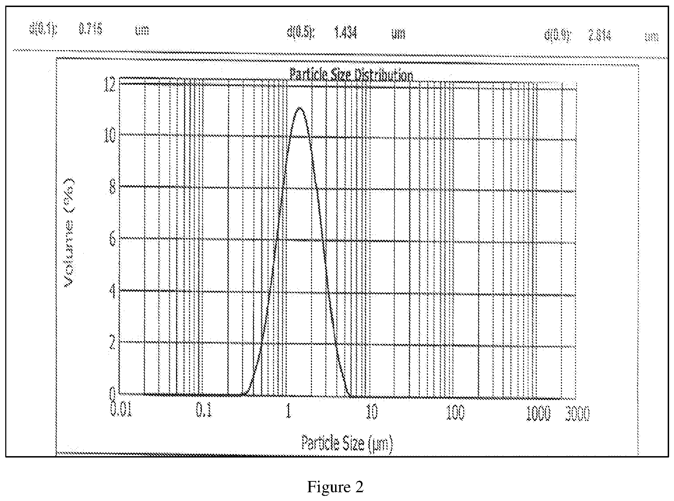

Figure 2 is a histogram of Malvern Particle size analyzer wherein the Particle Size Distribution of nilotinib Butanesulfonate is depicted. (2:1); Dio=0.715 µms, D₅₀ =1.434 µms and D₉₀=2.9 µms.

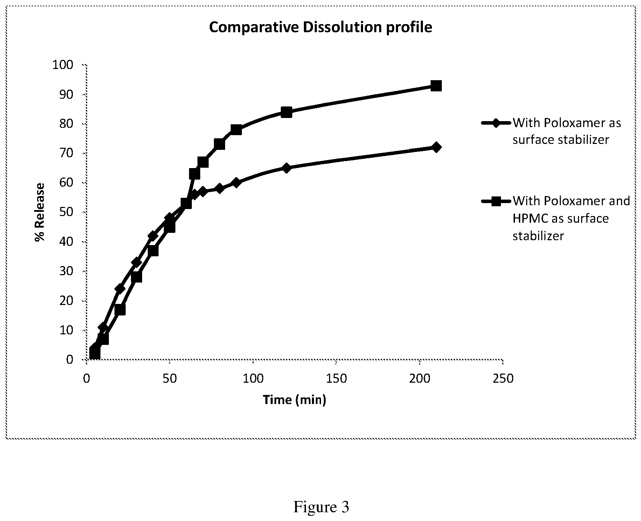

Figure 3: In vitro dissolution profile of two dosage forms of present invention containing 125 mg of nilotinib base, one containing poloxamer as surface stabilizer and another containing poloxamer and low viscosity hydroxypropyl methyl cellulose as surface stabilizer. The in vitro dissolution was carried out in 500 ml of 0.01N hydrochloride acid, Type I USP apparatus at 75 rpm, followed by addition of 100 mL of pH 4.5 Acetate buffer with ImM sodium taurocholate.
Figure 4: It is a comparative mean plasma nilotinib concentration Vs time profile after oral administration of dosage form of present invention in fasting condition, at a dose of 125 mg of nilotinib as nilotinib butanedisulphonate (2:1) and reference, Tasigna^{®} containing 200 mg of nilotinib as nilotinib hydrochloride.

### DESCRIPTION OF THE INVENTION

Present invention provides a pharmaceutical dosage form having a composition comprising nilotinib butanedisulphonate (2:1) or nilotinib butanedisulphonate (1:1) for use in treating resistant or intolerant Philadelphia chromosome positive chronic myelogenous leukaemia, comprising orally administering to a patient in need thereof daily doses of nilotinib in the range from 300 mg to 500 mg.

In one preferred embodiment of the present invention, the daily dose of nilotinib is in the range from 400 mg to 500 mg.

The nilotinib butanedisulfonate used in the composition of the dosage form according to the present invention is selected from nilotinib 1.4 butanedisulfonate (2:1), nilotinib butanedisulfonate (1:1) or hydrates, anhydrates thereof, wherein, 1:1 or 2:1 refers to mole ratio of nilotinib to butanesulfonic acid. In preferred embodiments, the nilotinib is in the form of its butanedisulfonic acid salt (2:1).

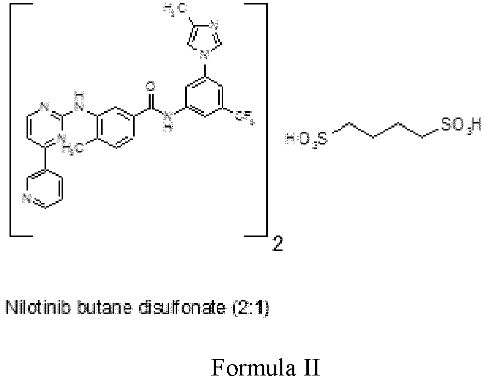

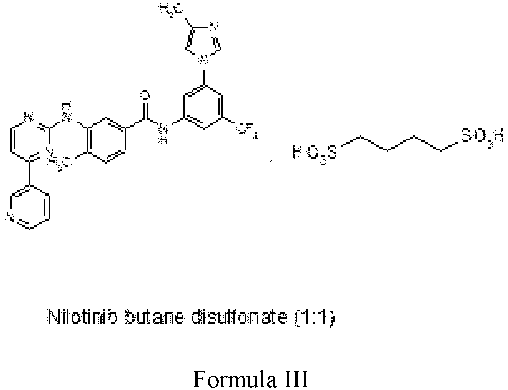

Preferably, when the daily dose of nilotinib is 300 mg, two unit dosage forms, each comprising 75 mg of nilotinib are orally administered twice a day. In one embodiment, wherein the leukemia is resistant or intolerant Philadelphia chromosome positive chronic myelogenous leukemia, the daily dose of nilotinib is in the range from 300 to 500 mg, wherein two unit dosage forms each unit dosage form comprising 75 to 125 mg of nilotinib are orally administered twice a day. Preferably, wherein the leukemia is resistant or intolerant Philadelphia chromosome positive chronic myelogenous leukemia the daily dose of nilotinib is 400 mg, wherein two unit dosage forms, each unit dosage form comprising 100 mg of nilotinib are orally administered twice a day. In another embodiment wherein the leukemia is resistant or intolerant Philadelphia chromosome positive chronic myelogenous leukemia, the daily dose of nilotinib is in the range from 300 to 500 mg, wherein a single unit dosage form comprising 150 to 250 mg of nilotinib is orally administered twice a day. Preferably, wherein the leukemia is resistant or intolerant Philadelphia chromosome positive chronic myelogenous leukemia the daily dose of nilotinib is 400 mg, wherein a single unit dosage form comprising 200 mg of nilotinib is orally administered twice a day.

In another embodiment, the present invention in one embodiment provides a dosage form for use in treating leukaemia comprising orally administering on an empty stomach or in the fed state to a patient in need thereof daily doses of nilotinib in the range from 300 mg to 500 mg, wherein the nilotinib is orally administered in a dosage form having a composition comprising nilotinib 1,4 butanedisulphonate (2:1) or nilotinib butanedisulphonate (1:1), wherein the leukemia is leukemia is resistant or intolerant Philadelphia chromosome positive chronic myelogenous leukemia. In these embodiments, the daily dose of nilotinib is in the range from 300 to 500 mg, wherein two unit dosage forms each unit dosage form comprising 75 to 125 mg of nilotinib are orally administered twice a day on an empty stomach or in the fed state. Preferably, the daily dose of nilotinib is 400 mg, wherein two unit dosage forms each unit dosage form comprising 100 mg of nilotinib are orally administered twice a day, on an empty stomach or in the fed state. In another embodiment wherein the leukemia is resistant or intolerant Philadelphia chromosome positive chronic myelogenous leukemia, the daily dose of nilotinib is in the range from 300 to 500 mg, wherein a unit dosage form comprising 150 to 250 mg of nilotinib is orally administered twice a day. Preferably, wherein the leukemia is resistant or intolerant Philadelphia chromosome positive chronic myelogenous leukemia the daily dose of nilotinib is 400 mg, wherein a unit dosage form comprising 200 mg of nilotinib is orally administered twice a day, wherein dosage form is administered on an empty stomach or in the fed state.

The present invention in one aspect provides a dosage form of which is in the form of microparticles. The microparticles of nilotinib butanedisulphonate (2:1) or nilotinib butanedisulphonate (1:1) may have particle size distribution characterized in that the D₉₀ is less than 25 microns. Alternatively, the The microparticles of nilotinib butanedisulphonate (2:1) or nilotinib butanedisulphonate (1:1) may have particle size distribution characterized in that the D₉₀ is less than 10 microns or less than 5 microns or less than 3 microns. In one specific embodiment, the nilotinib butanedisulphonate (2:1) used in the composition of the dosage form is crystalline having a powder X-ray diffraction pattern having powder X-ray diffraction peaks at 5.9, 8.1, 26.3 and 26.9 ± 0.2 degrees 2-theta. In one specific embodiment, the composition of the dosage form of the present invention contains a surface stabilizer. Also, for instance, in another embodiment of the present invention for treatment of resistant or intolerant Philadelphia chromosome positive chronic myelogenous leukemia, the total daily dose is in the range from 300 to 500 mg, more particularly 400 mg instead of currently prescribed 800 mg daily dose.

The present invention further provides a pharmaceutical dosage form having a composition comprising nilotinib butanedisulphonate (2:1) or nilotinib butanedisulphonate (1:1) for use in the treatment of newly diagnosed Philadelphia chromosome positive chronic myeloid leukemia at a total daily dose in the range from 300 mg to 375 mg. In one embodiment of the invention for treatment of newly diagnosed Philadelphia chromosome positive chronic myeloid leukemia the total daily dose is in the range from 300 mg to 375 mg instead of currently prescribed 600 mg daily dose of nilotinib base.

The dosage form of the present invention, comprises a composition comprising nilotinib butanedisulphonate (2:1) or (1:1) and pharmaceutically acceptable excipient. The nilotinib butandisulphonate (2:1) or (1:1) may be present in the form of micro-particles. In one embodiment, it is present in a crystalline form and in another; it is present in an amorphous form. In another aspect of the present invention, the nilotinib butanedisulphonate (2:1) is used. In one specific embodiment, the nilotinib butanedisulphonate (2:1) is in the hydrous form having less than 5 % of water content, preferably in the range of 3 to 8 % as determined by Karl Fischer technique. In another embodiment, the nilotinib butanedisulphonate (2:1) is in the anhydrous form. In one aspect, nilotinib butanedisulfonate (1:1) is used. In certain embodiments, crystalline nilotinib butanedisulphonate (2:1) is used. Various polymorphic forms of nilotinib butanedisulphonate (2:1) can be used. For example, a crystalline Form II having an X-ray powder diffraction pattern comprising characteristic peaks at 5.9, 8.1, 26.3 and 26.9 ± 0.2 degrees 2θ. It is possible to use a crystalline Form III of 2:1 salt of nilotinib with butanedisulfonic acid having water content of less than 1 % in which the X-ray powder diffraction pattern comprising characteristic peaks at 10.0, 20.3, 20.9 and 25.2 ± 0.2 degrees 2θ. The dosage form can have nilotinib butanedisulfonic acid (1:1). It can be present in a crystalline Form IV having an X-ray powder diffraction pattern comprising characteristic peaks at 5.9, 8.1, 26.3 and 26.9 ± 0.2 degrees 2θ. The crystalline Form IV of the nilotinib butanedisulfonic acid (1:1) can have additional characteristic peaks at 19.6, 21.1, 21.9 and 27.5 ± 0.2 degrees 2θ.

The nilotinib nilotinib butanedisulphonate (2:1) or (1:1) nilotinib butanedisulphonate (2:1) or (1:1) used in the composition of the dosage form of the present invention may be in the microparticle or micronized form such that it has a particle size distribution having D₉₀ less than 10 microns, wherein the 'D₉₀ of 10 microns' means 90% of the particles have a particle size of less than 10 microns. In several other embodiments, crystalline nilotinib butanedisulphonate (2:1) has a D₉₀ of less than 3 microns or 7.5 microns or 10 microns, 15 microns or 25 microns, respectively. In these embodiments, the D₅₀ ranges from less than 1.5 microns, less than 3.5 microns, less than 6 microns or less than 8 microns or less than 12 microns, respectively. In another aspect of the invention, the nilotinib butanedisulphonate (1:1) is used. For example, the D₉₀ of particles of nilotinib butanedisulphonate (1:1) is less than 3 microns, or less than 2 microns or less than 1 micron. In these cases, the D₅₀ of particles of nilotinib butanedisulphonate is less than 2 microns, less than 1.5 microns or less than 0.5 microns. In such embodiments, the nilotinib butanedisulphonate may be crystalline, semicrystalline or amorphous. In certain embodiments, it is possible to include a mixture of microparticles and nanoparticles of either nilotinib butanedislphonate (2:1) or nilotinib butanedisulphonate (1:1). In certain embodiments, the particles of nilotinib butanedisulphonate (2:1) or (1:1) may be adsorbed onto or associated with the surface thereof a non-crosslinked surface stabilizer. Suitable surface stabilizers include, but are not limited to, known organic and inorganic pharmaceutical excipients. Such excipients include various polymers, low molecular weight oligomers, natural products, and surfactants. Surface stabilizers include nonionic, ionic, anionic, cationic, and zwitterionic surfactants. The polymeric surface stabilizers suitable according to the present invention are the ones that provide low viscosity when tested as 2 % by weight solution in aqueous medium. Suitable grades of the polymer are those that provide viscosity of less than 20 centipoise. Particularly, these polymeric surface stabilizers are the ones that do not interfere or retard the release of the drug, when orally administered.

In one embodiment of the invention, the composition of the dosage form comprises a surface stabilizer which is a povidone polymer. Representative examples of surface stabilizers include hydroxypropyl methylcellulose (now known as hypromellose), albumin, hydroxypropylcellulose, polyvinylpyrrolidone, sodium lauryl sulfate, dioctylsulfosuccinate, gelatin, casein, lecithin (phosphatides), dextran, gum acacia, cholesterol, polyoxyethylene castor oil derivatives, polyoxyethylene sorbitan fatty acid esters (e.g., the commercially available Tweens^{®} such as e.g., Tween^{®} 20 and Tween^{®} 80 (ICI Speciality Chemicals)); polyethylene glycols, polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminum silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (e.g., Pluronics^{®} F68 and F108, which are block copolymers of ethylene oxide and propylene oxide); poloxamines (e.g., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-IOG^{®} or Surfactant 10-G^{®} (Olin Chemicals, Stamford, Conn.); Crodestas SL-40^{®} (Croda, Inc.); and SA90HCO, which is C₁₈H₃₇CH₂C(O)N(CH₃)-CH₂(CHOH)₄(CH₂O H)₂ (Eastman Kodak Co.); decanoyl-N-methylglucamide; n-decyl (-D-glucopyranoside; n-decyl (-D-maltopyranoside; n-dodecyl (-D-glucopyranoside; n-dodecyl (-D-maltoside; heptanoyl-N-methylglucamide; n-heptyl-(-D-glucopyranoside; n-heptyl (-D-thioglucoside; n-hexyl (-D-glucopyranoside; nonanoyl-N-methylglucamide; n-noyl (-D-glucopyranoside; octanoyl-N-methylglucamide; n-octyl-(-D-glucopyranoside; octyl (-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like. Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate. Other useful cationic surface stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C12-15dimethyl hydroxyethyl ammonium chloride or bromide, coconut dimethyl hydroxyethyl ammonium chloride or bromide, myristyl trimethyl ammonium methyl sulfate, lauryl dimethyl benzyl ammonium chloride or bromide, lauryl dimethyl (ethenoxy)4 ammonium chloride or bromide, N-alkyl (C12-18) dimethylbenzyl ammonium chloride, N-alkyl (C14-18)dimethyl-benzyl ammonium chloride, N-tetradecylidmethylbenzyl ammonium chloride monohydrate, dimethyl didecyl ammonium chloride, N-alkyl and (C12-14) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C12-14) dimethyl 1-naphthylmethyl ammonium chloride and dodecyldimethylbenzyl ammonium chloride, dialkyl benzenealkyl ammonium chloride, lauryl trimethyl ammonium chloride, alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar. Nonpolymeric surface stabilizers are any non-polymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound. In embodiments wherein a surface stabilizer is used, a combination of more than one surface stabilizer can be used in the composition of the dosage form of the present invention. Another embodiment of this invention provides a dosage form having composition comprising a nilotinib butanedisulfonate (2:1) or nilotinib butanedisulfonate (1:1), at least one pharmaceutically acceptable excipient, and at least one pharmaceutically acceptable solubilizer.

Representative solubilizers include, but are not limited to, polyvinyl caprolactam-polyvinyl acetate-polyethylene glycol graft copolymer, commercially available as Soluplus^{®}, PUREBRIGHT mb-37-50T and PUREBRIGHT mb-37-100T (2-methacryloyloxyethyl phosphorylcholine (MPCTM) and n-butyl methacrylate (BMA)) copolymers, Kollicoat (PVA-PEG graft copolymer), poly(styrene-co-methyl methacrylate-co-maleic anhydride) and poly(ethylene oxide) monomethyl ether graft copolymer, poly(vinyl alcohol)-poly(ethylene glycol) graft copolymer and poly(N-isopropylacrylamide)-b-[poly(ethyl acrylate)-g-poly (2-vinylpyridine)], polyoxyethylene-polyoxypropylene block copolymer available as poloxamer 105, poloxamer 108, poloxamer 122, poloxamer 123, poloxamer 124 (mol.wt 2090-2360), poloxamer 181, poloxamer 188 (mol.wt 7680-9510), poloxamer 212, poloxamer 217, poloxamer 235, poloxamer 237 (mol wt 6840-8830), poloxamer 282, poloxamer 331, poloxamer 338 (mol wt 12700-17400), poloxamer 401, poloxamer 403 and poloxamer 407 (mol wt 9840-14600) or mixtures thereof. In another embodiment, a block copolymer may be selected from poly(vinyl alcohol) and poly(ethylene glycol) copolymer, poly(methyl methacrylate) and poly(ethylene glycol), poly(methylmethacrylate) and poly(methacrylic acid), poly(lactic acid) and poly(ethylene glycol), Poly(ethyleneoxide)-poly(butadiene), Poly(ethyleneoxide)-Poly(ethylene ethylene), 2-methacryloyloxyethyl phosphorylcholine (MPC^{™}) and n-butyl methacrylate (BMA, poly(2-(dimethylamino)ethyl methacrylate) (PDMAEMA) and poly(methyl methacrylate) (PMMA), Dextran-block-poly(s-caprolactone) (DEX-b-PCL) (amphiphilic diblock copolymer), Poly Vivo mPEG-PLGA diblock copolymers, PolyVivo mPEG-PCL diblock copolymer, PLGA-block-PEG-block-PLGA) - (poly(lactic acid-co-glycolic acid)-block-poly(ethylene glycol)-block-poly(lactic acid-co-glycolic acid) triblock copolymers, mPEG-PLLA (Methoxy poly(ethylene glycol)-b-poly(L-lactide)) diblock copolymers, mPEG-PDLLA (Methoxy poly(ethylene glycol)-b-poly(D,L-lactide)) diblock copolymers, mPEG-PS (Methoxy poly(ethylene glycol)-b-poly(styrene)) diblock copolymers, mPEG-P(5BZTMC) (Methoxy poly(ethylene glycol)-poly(5-benzyloxy-trimethylene carbonate)) copolymers, mPEG-PTMC (Methoxy poly(ethylene glycol)-poly(trimethylene carbonate)) copolymers, PCL-PEG-PCL (Poly(caprolactone)-b-poly(ethylene glycol)-b-poly(caprolactone)) copolymers, PLA-PCL-PEG-PCL-PLA (Poly(D,L) lactide-b-Poly(caprolactone)-b-Poly(ethylene glycol)-b-Polycaprolactone-b-Poly(D,L)lacide) copolymers, PLA-PEG-PLA (Poly(D,L-lactide)-b-poly(ethylene glycol)-b-poly(D,L-lactide)) copolymers, PDLLA-PEG-PDLLA (Poly(D,L-lactide)-b-poly(ethylene glycol)-b-poly(D,L-lactide)) copolymers, PEG-PDLLA-Decyl(Poly(ethylene glycol)-b-poly(D,L-lactic acid)-decyl) copolymers, Kollidon VA 64 poly(vinylpyrrolidone-vinylacetate copolymer), Poly(N-vinyl-2-pyrrolidone)-poly(D,L-lactide), Poly(2-ethyl-2-oxazline)-block-(poly(epsilon-caprolactone) copolymer, Poly(ethylene oxide)-poly(butylene oxide) di and triblock copolymers, Polystyrene-poly(ethylene oxide) di and triblock copolymers, Poly(2-methyl-2-oxazoline)-b-poly(2-alkyl-2-oxazoline), Poly(methyl methacrylate)-poly(ethylene oxide) di block copolymer, Poly(N-isopropyl acrylamide)-Poly(y-benzyl-L-glutamate), Poly(ethylene oxide)-Poly(methylidene malonate), Poly(ethylene oxide)-poly(acrylic acid), Poly(ethylene oxide)-poly(methacrylic acid), Poly(ethylene oxide)-poly(vinyl benzoate), Poly(ethylene oxide)-Poly(N-isopropylacrylamide), Poly(ethylene oxide)-Poly(2-vinyl pyridine), Poly(vinyl benzyl alcohol)-Poly(oligo(ethylene glycol)methacrylate), Polystyrene-poly(acrylic acid), Polystyrene-poly(methacrylic acid) and Poly(2-ethoxyethyl vinyl ether)-Poly(2-methoxy ethyl vinyl ether), polyoxyethylene ethers, polyoxyethylene fatty acid esters, polyethylene glycol fatty acid esters, polyoxyethylene hydrogenated castor oil, polyoxyethylene alkyl ether, polysorbates, and or mixtures and combinations thereof. In certain embodiments, the solubilizer is a poloxamer. In some such embodiments, the poloxamer is poloxamer 407.

In some embodiments, composition of the dosage form comprises from about 0.1% to about 30% by weight of solubilizer, based upon total weight of the composition of the dosage form. The stabilizers are preferably adsorbed onto or associated with the surface of the nilotinib butanedisulphonate particles. Stabilizers useful herein do not chemically react with the nilotinib butanedisulphonate particles or itself. In another embodiment, the compositions of the dosage form can comprise two or more surface stabilizers. In a preferred embodiment, each solid unit dosage form comprises of nilotinib 1,4 butanedisulphonate (2:1) or nilotinib burtanedisulphonate (1:1) in the range of 25 mg to 150 mg wherein dosage form is in the form of hard gelatin capsule and two units are administered twice daily. In another preferred embodiment, the composition present in the solid dosage form contains 25 mg to 150 mg of nilotinib in the form of crystalline butanedisulphonate (2:1) having a XRD pattern of a with characteristic XRD peaks at 5.9, 8.1, 26.3 and 26.9 ± 0.2 2θ values and having a particle size distribution such that D₁₀=0.7 3 µm, D₅₀ is less than 1.4 µm and D90 is 3 µm and one or more surface stabilizers such as polyoxyethylene-polyoxypropylene block copolymer in amount ranging from 0.01 to 10 % by weight, or hydroxypropylmethyl cellulose low viscosity (15 cps), present in amounts ranging from 10 to 25 % by weight of the solid dosage form and pharmaceutically acceptable excipients such as disintegrants, lubricants and diluents. In one preferred embodiment, the composition is free of an organic acid such as citric acid, tartaric acid or the like.

The dosage form of the present invention may be in the form of capsules, tablets, pills, powders, and granules. In such solid dosage forms, the nilotinib butanedisulphonate, either (2:1) or (1:1) is admixed with at least one of the following: (a) one or more inert excipients (or carriers), such as sodium citrate or dicalcium phosphate; (b) fillers or extenders, such as starches, lactose, sucrose, glucose, mannitol and silicic acid; (c) binders, such as carboxymethylcellulose, alginates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, crospovidone and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; (j) glidants such as colloidal silicon dioxide, talc and (k) lubricants, such as talc, calcium stearate, magnesium stearate, solid polyethylene glycols, sodium lauryl sulfate, or mixtures thereof.

The composition of the dosage form of the present invention may be prepared by conventional methods such as dry granulation, wet granulation to obtain granules which may be filled into capsules, or compressed into tablets. It is also possible to blend therapeutically effective amounts of nilotinib butanedisulphonate (2:1) or nilotinib butanedisulphonate (1:1) with other excipients and the powder blend may be filled into hard gelatin capsules or compressed into a tablet. Alternatively, in other embodiments of this invention, the composition of the dosage form comprises a solid dispersion of nilotinib butanedisulfonate, at least one pharmaceutically acceptable excipient, and at least one pharmaceutically acceptable solubilizer. It may be prepared by a process, comprising the steps of a) preparing a homogeneous melt of nilotinib butanedisulphonate, said at least one one solubilizer and pharmaceutically acceptable excipient, and allowing the melt to solidify to obtain a solid dispersion product. In one preferred embodiment, the composition used in the dosage form comprises nilotinib butanedisulphonate (2:1) having a particle size distribution such than D₉₀ is less than 5 microns and D₅₀ is less than 2 microns, and one or more solubilizer such as triblock polymers of ethylene oxide and propylene oxide blocks in amount of 0.1 to 10 % by weight.

The dosage form of the present invention has an enhanced oral bioavailability when administered orally on empty stomach or administered in the fed state. Embodiments of the invention provide an increase in the extent of oral absorption by from about 25 % to 60% as compared to the commercially available dosage form containing 200 mg of nilotinib base as hydrochloride salt available as Tasigna^{®}. Thus the dosage form of the present invention is useful in the treatment of leukaemia at reduced daily doses of nilotinib.

Hereinafter, the invention will be more specifically described with reference to examples.

### EXAMPLE 1

Pharmaceutical composition in the form of capsule dosage form of nilotinib butanedisulfonate, are made as per the formula below.

**Table 1: composition details**

| Sr. No. | Ingredients | % by weight |
|---|---|---|
| 1 | Nilotinib butanedisulfonate (2:1) | 25.0 to 75 |
| | D₁₀= 0.715 microns, D₅₀= 1.434 microns and D₉₀ = 2.9 ums | |
| 2 | Poloxamer (Surface stabilizer) | 0.01 to 10 |
| 3 | Crospovidone (Disintegrant) | 1 to 10 |
| 4 | Lubricants | 0.25 to 3.0 |
| 5 | Lactose (Diluent) | q.s |

Excipients of the granular phase and the micronized nilotinib butanedisulphonate are blended and then formed in to granules by conventional granulation method. Granules are blended with extra-granular excipients and filled into capsules. In alternative examples, the composition is made by wet granulation and the granules filled into capsules. Alternatively the granules can also be compressed into tablets.

**Table 2: in vitro dissolution results of the solid dosage form**

| Time in minutes | Composition with poloxamer as surface stabilizer | Composition with poloxamer as surface stabilizer and hydroxypropyl methylcellulose |
|---|---|---|
| | % nilotinib released | |
| 5 | 4 | 2 |
| 10 | 11 | 7 |
| 20 | 24 | 17 |
| 30 | 33 | 28 |
| 40 | 42 | 37 |
| 50 | 48 | 45 |
| 60 | 53 | 53 |
| 65 | 56 | 63 |
| 70 | 57 | 67 |
| 80 | 58 | 73 |
| 90 | 60 | 78 |
| 120 | 65 | 84 |
| 210 | 72 | 93 |

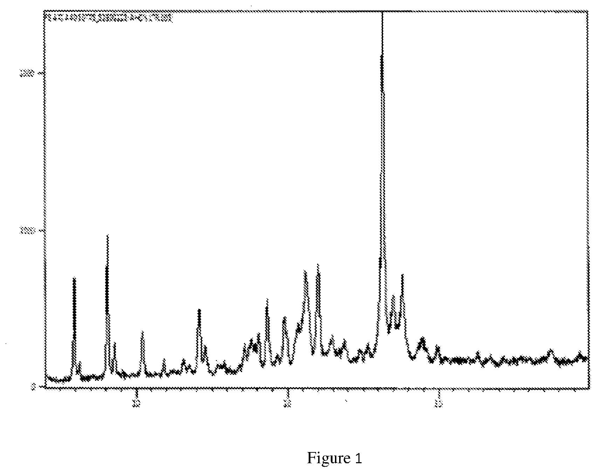

The composition in the form of capsule dosage form of example 1 was subjected to oral bioavailability determination. A randomized, open label two treatment, three period, three sequence, single dose reference replicated cross over relative bioavailability study under fasting conditions. Eighteen healthy human volunteers were dosed for the study and sixteen of them completed all the three period of the study. Particularly, the volunteers were overnight fasted for at least 10 hours after which they swallowed single dose of the composition of the present invention which contained micronized nilotinib butanedisulphonate in amounts equivalent to 125 mg of nilotinib (having particle size distribution of D₁₀= 0.715 microns, Dso= 1.434 microns and D₉₀ = 2.9 ums and XRD pattern as given in Figure 1) and the commercially available capsules available under the brand name Tasigna^{®} containing nilotinib hydrochloride in amounts equivalent to 200 mg of nilotinib. In each of the study period, blood samples were collected and nilotinib in plasma was quantified using a LCMS.MS method. The extent of absorption of the drug is determined by measuring the area under the curve at infinity AUC _{(0-∞)}.

It was found that the dosage form, intended for use in the method of the present invention, containing nilotinib butanedisulphonate in amounts equivalent to 125 mg of nilotinib) administered under fasting condition when compared with Tasigna^{®} (Nilotinib) 200 mg Capsules showed absorption of comparable amounts of nilotinib as measured by AUC_{(0-∞)}. The observed geometric mean values for AUC_{(0-inf)} were 11196.5 ng.hr/ml for nilotinib 125 mg dosage form for use in the method of the present invention and 11609.9 ng.hr/ml for Tasigna^{®} (Nilotinib) 200 mg Capsules. It is evident therefore that the dosage form used in the method of the present invention had significantly enhanced bioavailability. The present invention thus enables reduction of the dose of nilotinib.

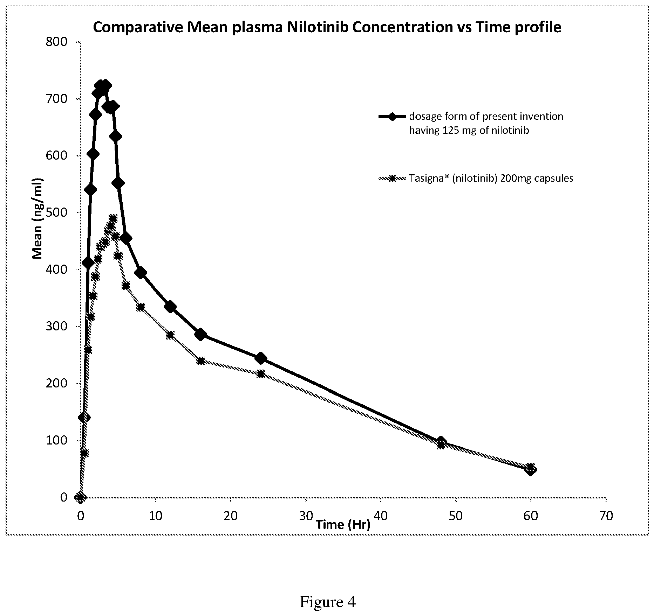

The composition of the unit dosage form of the present invention having both hydroxypropyl methyl cellulose and poloxamer, as surface stabilizer which contained nilotinib butanedisulphonate in an amount equivalent to 125 mg of nilotinib was subjected to the relative bioavailability study with Tasigna^{®} 200 mg as reference product. The mean plasma concentration-time profile is given in figure 4. The results are provided in Table 3.

**Table 3: Results of the oral bioavailability study**

| Product /Parameter → ↓ | AUC_{0-∞} ng.hr/ml (Least square mean) | Cₘₐₓ in ng/ml |
|---|---|---|
| Composition of the present invention (125 mg nilotinib) | 14669.23 | 754.72 |
| Tasigna^{®} (200 mg Nilotinib) | 12220.29 | 499.07 |

It was observed that even at a content of 125 mg nilotinib per unit solid dosage form, the composition of the present invention provided higher AUC_{0-∞} and Cₘₐₓ values. Tasigna^{®} is known to be effective, and therefore the above data shows that effective plasma levels can be obtained with solid dosage form of the present invention that contains a reduced dose of nilotinib.

## Claims

1. A pharmaceutical dosage form having a composition comprising nilotinib butanedisulphonate (2:1) or nilotinib butanedisulphonate (1:1) for use in treating resistant or intolerant Philadelphia chromosome positive chronic myelogenous leukaemia, comprising orally administering to a patient in need thereof daily doses of nilotinib in the range from 300 mg to 500 mg.

2. A pharmaceutical dosage form having a composition comprising nilotinib butanedisulphonate (2:1) or nilotinib butanedisulphonate (1:1) for use in the treatment of newly diagnosed Philadelphia chromosome positive chronic myeloid leukemia at a total daily dose in the range from 300 mg to 375 mg.

3. The pharmaceutical dosage form for use as claimed in claim 1 wherein the total daily dose is in the range from 400 mg to 500 mg .

## Patentansprüche

1. Pharmazeutische Dosierungsform, die eine Zusammensetzung aufweist, die Nilotinibbutandisulfonat (2:1) oder Nilotinibbutandisulfonat (1:1) umfasst, zur Benutzung in der Behandlung resistenter oder intoleranter Philadelphia-Chromosom-positiver chronischer myeloischer Leukämie, welche das orale Verabreichen täglicher Dosen von Nilotinib im Bereich von 300 mg bis 500 mg an einen Patienten, der dessen bedarf, umfasst.

2. Pharmazeutische Dosierungsform, die eine Zusammensetzung aufweist, die Nilotinibbutandisulfonat (2:1) oder Nilotinibbutandisulfonat (1:1) umfasst, zur Benutzung in der Behandlung neu diagnostizierter Philadelphia-Chromosompositiver chronischer myeloischer Leukämie in einer täglichen Gesamtdosis im Bereich von 300 mg bis 375 mg.

3. Pharmazeutische Dosierungsform zur Benutzung nach Anspruch 1, wobei die tägliche Gesamtdosis im Bereich von 400 mg bis 500 mg ist.

## Revendications

1. Forme posologique pharmaceutique ayant une composition comprenant du butanedisulfonate de nilotinib (2:1) ou du butanedisulfonate de nilotinib (1:1) pour une utilisation dans le traitement de la leucémie myéloïde chronique à chromosome Philadelphie positif résistante ou intolérante, comprenant l'administration par voie orale à un patient qui le nécessite de doses quotidiennes de nilotinib dans la plage de 300 mg à 500 mg.

2. Forme posologique pharmaceutique ayant une composition comprenant du butanedisulfonate de nilotinib (2:1) ou du butanedisulfonate de nilotinib (1:1) pour une utilisation dans le traitement de la leucémie myéloïde chronique à chromosome Philadelphie positif nouvellement diagnostiquée à une dose quotidienne totale dans la plage de 300 mg à 375 mg.

3. Forme posologique pharmaceutique pour une utilisation selon la revendication 1, dans laquelle la dose quotidienne totale est dans la plage de 400 mg à 500 mg.
